# EUROPÄISCHE PATENTSCHRIFT

(11) **EP 2 122 338 B1**
(45) Veröffentlichungstag und Bekanntmachung des Hinweises auf die Patenterteilung: **07.04.2010**
(21) Anmeldenummer: 08715499.3
(22) Anmeldetag: 14.02.2008
(51) Int. Cl.: G01N 27/413, G01N 27/49, C09K 5/06, G01N 33/00

(54) **OZONSONDE MIT HYDROTHERMISCHEM PUFFER**
OZONE SENSOR HAVING A HYDROTHERMAL BUFFER
SONDE D'OZONE À TAMPON HYDROTHERMIQUE

(30) Priorität: 21.02.2007 DE 102007009377
(43) Veröffentlichungstag der Anmeldung: 25.11.2009
(73) Patentinhaber: Stiftung Alfred-Wegener-Institut für Polar- und Meeresforschung, 27570 Bremerhaven (DE)
(72) Erfinder: ZÖLLNER, Mathias, 99310 Wipfratal (DE)
(86) Internationale Anmeldenummer: PCT/DE2008/000286
(87) Internationale Veröffentlichungsnummer: WO 2008/101478

(56) Entgegenhaltungen:
- WO-A-2005/097325
- US-A- 3 681 228
- US-B1- 6 298 907
- JOHNSON B J ET AL: "Electrochemical concentration cell (ECC) ozonesonde pump efficiency measurements and tests on the sensitivity to ozone of buffered and unbuffered ECC sensor cathode solutions" JOURNAL OF GEOGRAPHICAL RESEARCH, RICHMOND, VA, US, Bd. 107, Nr. D19, 16. Oktober 2002 (2002-10-16), Seiten ACH8.1-ACH8.18, XP009100124 ISSN: 0148-0227 in der Anmeldung erwähnt
- KOMHYR W D: "Electrochemical concentration cells for gas analysis" ANNALES DE GEOPHYSIQUE, PARIS, FR, Bd. 25, Nr. 1, 1. Januar 1969 (1969-01-01), Seiten 203-210, XP009100123 ISSN: 0003-4029 in der Anmeldung erwähnt
- "JOSIE Electrochemical Concentration Cell Ozone Sonde (ECC)"[Online] 13. Februar 2006 (2006-02-13), XP002484994 Gefunden im Internet: URL:http://www.fz-juelich.de/icg/icg-2/jos ie/ozone_sondes/ecc> [gefunden am 2008-05-13] in der Anmeldung erwähnt
- WANG ET AL: "Effect of phase-change material on energy consumption of intelligent thermal-protective clothing" POLYMER TESTING, ELSEVIER, Bd. 25, Nr. 5, 1. August 2006 (2006-08-01), Seiten 580-587, XP005585898 ISSN: 0142-9418

## Beschreibung

Die Erfindung bezieht sich auf eine Ozonsonde zur in situ Messung von stratosphärischen Ozonkonzentrationsprofilen mittels Ballonaufstiegen, umfassend eine Messzelle mit einer wässrigen Reaktionslösung mit einem von ihrem verfahrensbedingten Salzgehalt abhängigen Schmelz- und Siedepunkt, eine Pumpe zur Durchleitung von Umgebungsluft durch die wässrige Reaktionslösung, eine Stromversorgung, eine Einrichtung zur Registrierung des Reaktionsstroms und Übermittlung der Daten und eine Tragevorrichtung zur Anordnung aller Komponenten,

Die hauptsächlich heute zur Anwendung kommende Methode zur in situ Messung stratosphärischer Ozon-Konzentrationen basiert auf der Auswertung einer elektrochemischen Reaktion des Ozons bei der Durchleitung mittels einer Pumpe durch eine spezielle wässrige Reaktionslösung in einer Messzelle innerhalb einer ballongetragenen Sonde. Der Höhenbereich für sinnvolle Messungen mit diesem Verfahren ist dabei nach oben vor allem dadurch begrenzt, dass die Druck- und Temperaturbedingungen der Messumgebung schließlich zu einer Phasenumwandlung der wässrigen Reaktionslösung führen. Bereits eine teilweise Phasenumwandlung verändert die wässrige Reaktionslösung häufig derart, dass die Messung beendet werden muss. Eine prinzipielle physikalische Grenze ist dabei durch den Tripelpunkt der wässrige Reaktionslösung gesetzt. Sich dieser Grenze möglichst weit zu nähern, ist ein zu optimierendes Ziel bei jeder dieser teuren Messungen des besonders in der Stratosphäre so bedeutsamen Gases Ozon.

Die Relevanz dieses Problems ergibt sich aus der Frage, wie groß der Anteil der teuren Sondenaufstiege ist, bei denen die Flüssigphasenbedingungen verlassen werden und an welcher Stelle des Ozonprofils dies im Mittel erfolgt. Zur Relevanzanalyse standen Auswertungen von 878 Ozonsondenaufstiege der vergangenen vierzehn Jahre an der Neumayer-Station des Alfred-Wegener-Instituts für Polar- und Meeresforschung in der Antarktis zur Verfügung. Dabei ergab sich das nachfolgend beschriebene Bild. Bei etwa 75% der Aufstiege wurden Umgebungsbedingungen erreicht, die entsprechend den Druck- und Innentemperaturmessungen im Sondengehäuse zu einer wenigstens teilweisen Phasenumwandlung der wässrige Reaktionslösung führten. Dabei wurde in 22% der Fälle die Nullgradgrenze unterschritten. In den übrigen 53% der Fälle wurde die Siedegrenze beim jeweiligen Umgebungsdruck überschritten. Im Mittel geschah dies in einer Höhe, bei der noch etwa 30% der abgeschätzten Gesamtozonsäule über der Sonde lagen, woraus entsprechende Unsicherheiten im Messergebnis resultieren. Eine untere Abschätzung für die Anzahl der weltweit benötigten Ozonsonden ist durch die Anzahl der Datensätze gegeben, die beim World Ozone and Ultraviolet Radiation Data Centre (WOUDC) in Toronto eingehen. Im Zeitraum zwischen 2000 und 2005 waren das im Mittel rund 2100 Ozonprofile pro Jahr. Alle diese Profile wurden mit elektrochemischen Sensoren aufgenommen, etwa 80% der genannten Sonden sind vom Typ Electrochemical Concentration Cell (ECC).

### Stand der Technik

In der **Veröffentlichung I** (Forschungszentrum Jülich, Institut für Chemie und Dynamik der Geosphäre, Troposphäre, Projekt Josie, Ozonsonden, ECC-Sonde, im Internet unter der url http://www.fz-juelich.de/icg/icg-ii/josie/ozone sondes/ecc), Stand 18.01.2007) wird der Aufbau der Sonden SPC-6A der Firma Science Pump Corporation, Camden, New Jersey, USA und ENSCHZ der Firma EN-SCI Corporation, Boulder, Colorado, USA beschrieben. Beide Sonden sind in Aufbau und Funktion nahezu identisch und folgen der Entwicklung von Komhyr, W.D. (Ann.Geophys., 25, 203-210, 1969) aus dem Jahre 1969. Danach ist die ECC-Sonde eine elektrochemische Zelle mit je einer anodischen und einer kathodischen Kammer aus Teflon. Beide Kammern enthalten Platin-Netzelektroden, die unter wässrigen Kaliumjodid-Reaktionslösungen mit unterschiedlichen Konzentrationen stehen. Bei Kaliumjodid handelt es sich um das weiße, unter starker Abkühlung sehr leicht in Wasser lösliche Kalium-Salz der lodwasserstoffsäure. Durch die verfahrensbedingte lonenkonzentration ergeben sich spezielle Siede- und Schmelzpunkte für die wässrige Reaktionslösung. Diese sind druckabhängig, sodass sich entsprechende Funktionsverläufe einstellen. Im Tripelpunkt schneiden sich die beiden Funktionsverläufe. Die beiden Kammern der ECC sind über eine Ionenmembran verbunden, die Stromfluss zulässt, den Austausch der wässrigen Reaktionslösungen beider Kammern aber verhindert. Die ECC benötigt keine externe Stromversorgung, da sie ihre treibende Kraft aus dem Unterschied der Konzentrationen der wässrigen Reaktionslösung beider Kammern (z.B. 0,06 Mol/l = 1% KJ zu ca. 8,0 Mol/l = gesättigt) zieht. Eine nicht mit Ozon reagierende Probenpumpe aus Teflon treibt die ozonhaltige Umgebungsluft durch die kathodische Kammer mit der niedriger konzentrierten KJ-Reaktionslösung und erzeugt freies Jod (J₂). Die Reaktionsgleichung ist: 2KJ + O₃ +H₂O → 2KOH + J₂ + O₂. An der Kathode wird das J₂ durch Aufnahme von 2 Elektronen in J₂- umgewandelt, das wandert zur Anode in der Kammer mit der hohen KJ-Konzentration, gibt dort die beiden Elektronen wieder ab und wird wieder zu J₂. Die Abgabe und Aufnahme von Elektronen an den Elektroden sorgt für einen der Ozonkonzentration in der Umgebungsluft proportionalen Strom in dem an die Elektroden angeschlossenen Stromkreis. Die auftretenden Einflüsse von Temperatur und Druck der Umgebungsluft werden bei der Beschreibung der Sondenfunktion jedoch nicht berücksichtigt. Die ganze Sonde wird für den Ballonflug in eine Box aus z.B. Polystyrolschaum gesetzt.

In der **Veröffentlichung II** ("Electrochemical Concentration Cell (ECC) ozonesonde pump efficiency measurements and tests on the sensitivity to ozone buffered and unbuffered ECC sensor cathode solutions", Journal of Geophysical Research, Vol. 107, No. D19, 4393, 2002) wird beschrieben, welche verfälschenden Einflüsse die Leistung der Pumpe für die durch die wässrige Reaktionslösung zu leitende Umgebungsluft und die Menge des der wässrigen Reaktionslösung häufig beigefügten Phosphatpuffers auf das Messergebnis nehmen. Ein Einfluss von Temperatur und Druck der Umgebungsluft wird bei den hier vorgestellten Untersuchungen nicht berücksichtigt.

Aus der US 3,681,228 (Electrochemical Concentration Cell for Gas Analysis), von der die vorliegende Erfindung als dem nächstliegenden Stand der Technik ausgeht, ist eine praktisch ausgeführte ECC bekannt. Sie weist ein Zellengehäuse auf, das zwei die Höhe des Zellengehäuse fast gänzlich ausnutzende parallele und dicht beieinander liegenden große Bohrungen enthält, die die anodische und die kathodische Kammer bilden. Die beiden Kammern sind über ihren ganze vertikale Länge mit einer ionendurchlässigen Membran verbunden. Beide Kammern enthalten Elektroden, z.B. aus Platingaze, mit abgedichteten Anschlüssen nach außen und sind mit gleichartiger wässriger Reaktionslösung unterschiedlicher lonen-Konzentrationen bis etwa zur halben Höhe gefüllt. Eine weitere vertikale Bohrung ist mit der kathodischen Kammer ständig verbunden und dient im Falle der ortsveränderlichen Verwendung der Sonde als Vorratsbehälter für wässrige Reaktionslösung. Alle Kammern sind nach oben abgeschlossen und enthalten Entlüfungsöffnungen, die durch dünne Rohre, die nach unten im Luftraum oberhalb der wässrigen Reaktionslösung enden und oberhalb der Kammern nach oben verlängert sind. Sie dienen dem Druckausgleich beim Durchleiten der Umgebungsluft, beim Ausgasen der wässrigen Reaktionslösung bei Druckabnahme mit zunehmender Höhe des Einsatzes sowie der Abführung von Reaktionslösungsgas beim Sieden unter niedrigem Druck in großer Einsatzhöhe. Alle Bauteile sind aus einem mit der wässrigen Reaktionslösung und den zu bestimmenden Gasen nicht reaktionsfähigen Material, z.B. Teflon gefertigt. Mit der Erwähnung des möglichen Siedens in großen Höhen wird dieser Umstand zumindest erkannt. Eine Berücksichtigung des dadurch und gegebenenfalls auch durch Einfrieren der wässrigen Reaktionslösung möglicherweise eintretenden Effekts auf die Messergebnisse wird jedoch nicht beschrieben.

Die Einflüsse von Temperatur und Druck der Umgebungsluft in großen Einsatzhöhen der Sonde, und damit der Gefahr des Einfrierens bzw. Siedens der wässrigen Reaktionslösung auf die Messergebnisse sind aber bekannt, da verschiedene Methoden der Temperaturpufferung an kommerziell angebotenen Sonden eingesetzt werden, um den Einsatzbereich in die Höhe auszudehnen. Die wässrige Reaktionslösung hat zu Beginn der Messung gewöhnlich noch Raumtemperatur. Die kritische Temperatur am Tripelpunkt beträgt etwa 0° Celsius. Die Temperaturen in der Stratosphäre unterhalb der Höhe des Tripelpunktdruckes liegen etwa im Bereich zwischen -20° Celsius und -100° Celsius. Eine thermische Isolierung der Messzelle durch dickwandigen Leichtkunststoff ist die vom Hersteller der Messsonden bevorzugt bereitgestellte Methode, um eine verzögerte Abkühlung der wässrigen Reaktionslösung zu gewährleisten und sich so mehr oder minder gut der maximalen Messhöhe zu nahem. Die thermische Isolierung allein, die vor allem aus Gewichtsgründen in ihrer Stärke begrenzt ist, kann häufig und insbesondere unter winterlichen Bedingungen das Problem nicht lösen. Vorzeitige Eiskristallbildung in der wässrigen Reaktionslösung ist die Folge bereits vor dem Erreichen des Tripelpunktdruckes. Um dem vorzubeugen, wird häufig eine thermische Brücke zu einer ebenfalls in der Sonde befindlichen und Verlustwärme produzierenden Batterie zum Betrieb der Pumpe hergestellt. Versuche mit zusätzlichen elektrischen Heizelementen neben der Messzelle oder mit Heizelementen, die Wärme aus langsamen chemischen Reaktionen beziehen, gab es ebenfalls. Derartige Methoden bergen grundsätzliche Probleme der Regelbarkeit, die sich nicht ohne deutlich erhöhten Kostenaufwand und zusätzliches und damit die Aufstiegshöhe der Sonde reduzierendes Gewicht lösen lassen. Entsprechend wird vom Einsatz zusätzlicher Regelungstechnik für die Wärmeanbindung bei den oben genannten Methoden aus Platz-, Gewichts- und Kostengründen gewöhnlich abgesehen. Somit besteht insbesondere bei niedrigen Stratosphärentemperaturen weiterhin das Risiko frühzeitiger starker Auskühlung der Messzelle. Andererseits ist unter den Bedingungen einer wärmeren Stratosphäre verstärkt mit einer Überhitzung der wässrigen Reaktionslösung zu rechnen, was aufgrund des niedrigen Umgebungsdruckes in der Stratosphäre mit dem beginnenden Verdampfen der wässrigen Reaktionslösung die Messung ebenfalls derart verfälscht, dass sie abgebrochen werden muss.

### Aufgabenstellung

Die Aufgabe für die vorliegende Erfindung ist es daher, eine gattungsgemäße ECC (Electrochemical Concentration Cell) der eingangs beschriebenen Art bereitzustellen, die für den Messeinsatz in den Höhen der Stratosphäre geeignet ist, in denen gewöhnlich Sieden oder Einfrieren der wässrigen Reaktionslösung bereits eintritt, dabei aber größere Gewichtszunahmen und/oder Kosten für wärmedämmende oder Regelungsmaßnahmen vermeidet und gleichzeitig einfach handhabbar ist. Die erfindungsgemäße **Lösung** für diese Aufgabe ist dem Hauptanspruch zu entnehmen. Vorteilhafte Weiterbildungen werden in den Unteransprüchen beschrieben.

Die erfindungsgemäße Ozonsonde ist grundsätzlich dadurch gekennzeichnet, dass die Messzelle von einem hydrothermischem Puffer als einem mit Wasser befüllbaren Gefäß umfasst ist. Dabei umschließt das Gefäß die Messzelle vollständig und der Stand des Wassers im Gefäß erreicht mindestens den Stand der wässrigen Reaktionslösung in der Messzelle. Weiterhin hat die wässrige Reaktionslösung erfindungsgemäß durch ihren verfahrensbedingten Salzgehalt einen niedrigeren Schmelzpunkt und einen höheren Siedepunkt als das Wasser. Schließlich ist bei der erfindungsgemäßen Ozonsonde noch vorgesehen, dass bei Erreichen der Stratosphäre die Temperatur in der Messzelle über dem Schmelzpunkt oder unter dem Siedepunkt der wässrigen Lösung durch die thermodynamisch passiv bewirkte Zuführung oder den Entzug von Energie aus der wässrigen Reaktionslösung durch das Wasser bei dessen früherem Phasenübergang vor Erreichen des Tripelpunktes stabilisiert wird.

Bei der hier vorgestellten Erfindung handelt es sich somit um einen hydrothermischen Puffer, der in mehrfacher Hinsicht eine einfache und effektive Optimierung des Messbereiches der elektrochemischen Ozonsonde ECC in den größeren Einsatzhöhen der Stratosphäre ermöglicht und nur eine geringe Gewichtszunahme der Ozonsonde bewirkt. Als hydrothermischer Puffer wird hier im weiteren gerätetechnischen Sinne das die Messzelle umfassende Gefäß mit seiner Wasserfüllung und gegebenenfalls weiteren Bestandteilen verstanden. Als zusätzliche Wärmekapazität vermindert das Wasser des hydrothermischen Puffers einerseits die typischerweise auftretende vorzeitigen Auskühlung der wässrigen Reaktionslösung. Deutlich wichtiger ist aber andererseits der Effekt, dass bei der auftretenden Druck- und Temperaturänderung bei Erreichen größerer stratosphärischer Höhen das Wasser des hydrothermischen Puffers seine Phasengrenze früher erreicht als die wässrige Reaktionslösung. Die wässrige Reaktionslösung hat durch ihren verfahrensbedingten Salzgehalt einen niedrigeren Schmelzpunkt und einen höheren Siedepunkt als das Wasser des hydrothermischen Puffers. Im Auskühlungsfall schützt die freiwerdende Erstarrungswärme des Wassers des hydrothermischen Puffers die wässrige Reaktionslösung und stabilisiert die Temperatur. Im Überhitzungsfall schützt die vom Wasser des hydrothermischen Puffers aufgenommene Siedewärme die wässrige Reaktionslösung und stabilisiert ebenfalls die Temperatur. Je nach zu erwartenden Temperaturbedingungen kann das Wasser des hydrothermischen Puffers auch vortemperiert werden und so zusätzliche thermische Energie speichern.

Ausgehend von dem geschilderten, bei stratosphärischen Ozonmessungen häufig auftretenden Problem der Phasenumwandlung der wässrigen Reaktionslösung, liegt primär der Ansatz nahe, Veränderungen der Wärmeisolierung bzw. einen zusätzlichen Wärmeeintrag, beispielsweise über ein einfaches Wasserbad mit einer passiven, puffernden Wärmekapazität, vorzunehmen, wobei allerdings das Problem der Regelung dieser Maßnahmen in Verbindung mit den besonderen Einsatzbedingungen in der Stratosphäre zu neuen Schwierigkeiten und schwer akzeptablen Realisierungskompromissen führt, z.B. durch stromverbrauchende Heizelemente und Regelungskomponenten, die durch das gesamte Sondengewicht stark beeinflussende Batterien gespeist werden müssen. Die Frage nach physikalischen Mechanismen, die genutzt werden können, um die Temperatur der wässrigen Reaktionslösung so zu stabilisieren, dass diese möglichst lange flüssig bleibt, führten zur erfindungsgemäßen Idee des hydrothermischen Puffers. Die gezielte Nutzung der passiven thermodynamischen Eigenschaften von Wasser, um einen Energieaustausch bei optimaler Temperatur zu realisieren, ist dabei entscheidend. Für die Bauteile einer Ozonsonde, die häufig nach ihrer ungesteuerten Landung in der Umwelt verbleibt, sind außerdem Aspekte des Umweltschutzes besonders aufmerksam zu beachten. Bei der Verwendung des hydrothermischen Puffers zur Temperaturstabilisierung in Ozonsonden beschränkt sich der zusätzliche Eintrag in die Umwelt im Wesentlichen auf das Gefäß, welches entsprechend dünnwandig und aus umweltverträglichen Materialien gefertigt werden kann. Das Wasser des hydrothermischen Puffers selbst ist an sich bereits völlig umweltneutral.

Bei der erfindungsgemäßen Ozonsonde mit ihrem speziellen hydrothermischen Puffer geht es um den sehr speziellen Effekt der Ausnutzung von systemimmanenten, ohne weitere erforderliche Beeinflussung von Außen freiwerdenden oder aufgenommenen Energiedifferenzen (Erstarrungswärme/Verdampfungswärme) durch spezifische und vorzeitig im Wasser des hydrothermischen Puffers hervorgerufene Phasenumwandlungen. Diese vorzeitigen, d.h. grundsätzlich vor den Phasenumwandlungen der wässrigen Reaktionslösungen von selbst eintretenden Phasenumwandlungen basieren auf dem anderen Schmelz- und Siedepunktverhalten des Wassers gegenüber der wässrigen Reaktionslösung. Diese weist durch die für den Ozonnachweis erforderliche Zugabe einer Salzsubstanz gegenüber dem bei der Erfindung eingesetzten, zusatzfreien Wasser eine höhere lonenkonzentration und damit eine verzögerte Phasenumwandlung auf. Beim Wasser werden der Gefrier-/Schmelzpunkt und der Siedepunkt, die beide druckabhängig sind und sich im Tripelpunkt des Wassers schneiden, jeweils früher erreicht. Der durch die Erfindung genutzte natürliche Energietransport vor Erreichen des Tripelpunktes führt zu einer bedeutsam längeren Flüssighaltung der wässrigen Reaktionslösung und damit zu einer bedeutsam längeren möglichen Messzeit mit der erfindungsgemäßen Ozonsonde.

Zusammenfassung der entscheidenden Vorteile der erfindungsgemäßen Ozonsonde:
1. Das Wasser des hydrothermischen Puffers besitzt grundsätzlich als Wärmespeicher bereits eine außerordentlich hohe Wärmekapazität, weist also einen Speichereffekt auf.
2. Das Wasser des hydrothermischen Puffers hat einen höheren Schmelzpunkt als die wässrige Reaktionslösung in der Messzelle. Es friert damit im Unterkühlungsfall zuerst ein und setzt dabei Erstarrungswärme frei, welche die Temperatur in der Messzelle über dem Gefrierpunkt der wässrigen Reaktionslösung stabilisiert, sorgt also für einen Erwärmungseffekt.
3. Das Wasser des hydrothermischen Puffers hat einen niedrigeren Siedepunkt als die wässrige Reaktionslösung in der Messzelle. Es verdampft damit im Überhitzungsfall unter dem niedrigen Stratosphärendruck zuerst und nimmt dabei Verdampfungswärme auf, welche die Temperatur in der Messzelle unterhalb des Siedepunktes der wässrigen Reaktionslösung stabilisiert, sorgt also für einen Kühleffekt.
4. Das Wasser des hydrothermischen Puffers ist grundsätzlich umweltneutral, besonders funktionssicher, mit nur geringen Kosten verbunden und erhöht kaum das Gewicht des Gesamtsystems.

Gemäß einer besonders vorteilhaften Weiterentwicklung der Ozonsonde nach der Erfindung beträgt die Wassermenge zur Befüllung des Gefäßes 25 g bis 50 g und die Grundfläche des Gefäßes 10 cm² bis 20 cm². Das Wasser wird zusammen mit dem Gefäß, das die Messzelle umfasst, als hydrothermischer Puffer eingesetzt. Dabei kommt es darauf an, die geringe Wassermenge durch innigen Kontakt mit der Messzelle besonders gut zu nutzen. Mit der angegebenen Wassermenge und der Grundfläche des Gefäßes wird erreicht, dass die Flüssigkeitsstände in dem Gefäß und der Messzelle ungefähr gleich hoch sind. Dadurch wird die geforderte Innigkeit des Kontakts zwischen den Flüssigkeiten mit einer optimal geringen Wassermenge des hydrothermischen Puffers in Bezug auf die durch die Konstruktion der Messzelle vorgegebenen Menge wässriger Lösung erreicht.

Es ist weiterhin besonders vorteilhaft, wenn das Gefäß der Ozonsonde eine zumindest teilweise Füllung mit porösem Material aufweist. Derartiges Material nimmt das Wasser des hydrothermischen Puffers auf und hält es in seinen Poren fest. Es verhindert einerseits nicht den innigen Kontakt des Wassers mit der Messzelle, sorgt aber andererseits dafür, dass das Wasser bei turbulenten Bewegungen der Ozonsonde nicht aus dem Gefäß fließt. Darüber hinaus ist es besonders von Vorteil, wenn das poröse Material Zellstoff ist. Zellstoff ist ein organisches und damit biologisch abbaubares Material. Es weist alle notwendigen Eigenschaften auf, das Wasser des hydrothermischen Puffers zu binden und in Kontakt mit der Messzelle zu bringen. Ferner ist es umweltfreundlich und kann damit problemlos nach der ungesteuerten Rückkehr der Sonde zum Erdboden in der Umwelt verbleiben.

Weiterhin sind es besonders vorteilhafte Weiterentwicklungen der Ozonsonde nach der Erfindung, wenn das Material des Gefäßes Teflon oder Polyethylen oder wasserdicht beschichtete Pappe ist. Teflon ist ein sehr haltbares umweltneutrales Material, das auch für andere Teile der Ozonsonde eingesetzt wird. Es hat keinerlei umweltschädliche Wechselwirkungen mit Chemikalien der Luft, des Wassers und des Bodens. Da es auch nicht mit Ozon reagiert, bleiben durch seinen Einsatz alle Messungen unverfälscht. Andererseits verbleibt Teflon wegen seiner stabilen Eigenschaften auch sehr lange in der Umwelt nach der Rückkehr der Ozonsonde zum Erdboden. Wenn Messzelle und Gefäß aus einem Stück gefertigt werden sollen, ist Teflon das am besten geeignete Material. Bei getrennter Fertigungsweise kommen hingegen besser Polyethylen oder wasserdicht beschichtete Pappe für das Gefäß zum Einsatz, da diese Materialien deutlich schneller biologisch abgebaut werden und bei dem vorgesehenen Einmaleinsatz der Ozonsonden die Dauerstandfestigkeit des Materials des Gefäßes nur eine untergeordnete Rolle spielt.

### Ausführungsbeispiel

Eine Ausbildungsform der Ozonsonde nach der Erfindung wird nachfolgend zum weiteren Verständnis der Erfindung anhand der schematischen Figuren näher erläutert. Dabei zeigt die
- **Figur**: die Ozonsonde in perspektivischer Ansicht von der Vorderseite

Die **Figur** zeigt die Ozonsonde **OS** in perspektivischer Ansicht. An der Vorderseite **VS** einer Tragevorrichtung **TV** ist ein Gefäß **GE,** in diesem Beispiel aus Teflon, befestigt, das zusammen mit Wasser **WA** darin den hydrothermischen Puffer **HP** bildet. In dem Gefäß **GE** ist eine aus einem linken und einem rechten Einzelbehälter **EL,ER** bestehende Messzelle **MZ** angeordnet, deren Einzelbehälter **EL,ER** mit hier nicht weiter dargestellter wässriger Reaktionslösung **WL** unterschiedlicher Konzentrationen gefüllt ist. Aus Gründen des Druckausgleichs ist das Gefäß **GE** in diesem Ausführungsbeispiel nach oben gänzlich offen. Es enthält ferner zur Vermeidung des Auslaufens oder Überfließens des Wassers **WA** bei kurzzeitigem Kippen oder heftigen Bewegungen der Ozonsonde **OS** eine Füllung **FU** aus porösem Material **PM ,** in diesem Ausführungsbeispiel Zellstoff **ZS,** das die Bewegung des Wassers **WA** im Gefäß **GE** dämpft. Seitlich oberhalb der Messzelle **MZ** ist eine Pumpe **PU** an der Tragevorrichtung **TV** angeordnet, die über einen rechten Schlauch **SR** Umgebungsluft **UL** ansaugt und über einen linken Schlauch **SL** in den linken Einzelbehälter **EL** der Messzelle **MZ** einleitet. Beide Einzelbehälter **EL,ER** weisen kurze Entlüftungsschläuche **ES** auf. An der Rückseite **RS** der Tragevorrichtung **TV** ist eine Stromversorgung **SV** und eine Einrichtung zur Registrierung des Reaktionsstroms des Ozons der Umgebungsluft **UL** mit der wässrigen Reaktionslösung **WL** in der Messzelle **MZ** und Übermittlung der daraus gewonnenen Daten **RD** untergebracht. Elektrische Verbindungen sind nicht dargestellt.

Diese Ausführungsform des Gefäßes **GE** ist nur beispielhaft gewählt. Andere Behältnisse, die sich in Größe, Form und Material vom gezeigten Beispiel unterscheiden, z.B. auch flexible Gefäße, können ebenso für den hydrothermischen Puffer **HP** genutzt werden. Wichtig ist dabei aber vor allem die Realisierung eines ausreichenden thermischen Kontaktes des Wassers **WA** des hydrothermischen Puffers **HP** zur wässrigen Reaktionslösung **WL** in der Messzelle **MZ.** Das Gefäß **GE** muss also in jeder Ausführungsform die Messzelle **MZ** vollständig umschließen und der Stand des Wassers **WA** im Gefäß **GE** soll den der wässrigen Reaktionslösung **WL** in der Messzelle **MZ** mindestens erreichen.

### Bezugszeichenliste

- **EL**: linker Einzelbehälter
- **ER**: rechter Einzelbehälter
- **ES**: Entlüftungsschlauch
- **FU**: Füllung
- **GE**: Gefäß
- **HP**: hydrothermischer Puffer
- **MZ**: Messzelle
- **OS**: Ozonsonde
- **PM**: poröses Material
- **PU**: Pumpe
- **RD**: Einrichtung zur Registrierung und Datenübermittlung
- **RS**: Rückseite
- **SL**: linker Schlauch
- **SR**: rechter Schlauch
- **SV**: Stromversorgung
- **TV**: Tragevorrichtung
- **UL**: Umgebungsluft
- **VS**: Vorderseite
- **WA**: Wasser
- **WL**: wässrige Reaktionslösung
- **ZS**: Zellstoff

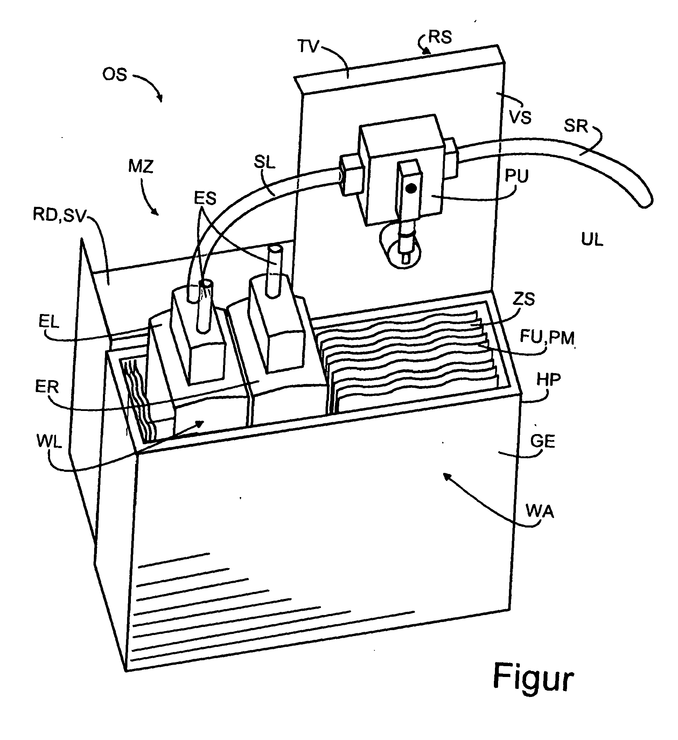

## Patentansprüche

1. Ozonsonde (OS) zur in situ Messung von stratosphärischen Ozonkonzentrationsprofilen mittels Ballonaufstiegen, umfassend eine Messzelle (MZ) mit einer wässrigen Reaktionslösung (WL) mit einem von ihrem verfahrensbedingten Salzgehalt abhängigen Schmelz- und Siedepunkt, eine Pumpe (PU) zur Durchleitung von Umgebungsluft (UL) durch die wässrige Reaktionslösung (WL), eine Stromversorgung (SV), eine Einrichtung zur Registrierung des Reaktionsstroms und Übermittlung der Daten (RD) und eine Tragevorrichtung (TV) zur Anordnung aller Komponenten,
**dadurch gekennzeichnet, dass**
die Messzelle (MZ) von einem hydrothermischem Puffer als einem mit Wasser (WA) befüllten Gefäß (GE) umfasst ist,
• wobei das Gefäß (GE) die Messzelle (MZ) vollständig umschließt und der Stand des Wassers (WA) im Gefäß (GE) den Stand der wässrigen Reaktionslösung (WL) in der Messzelle (MZ) mindestens erreicht,
• wobei die wässrige Reaktionslösung (WL) durch ihren verfahrensbedingten Salzgehalt einen niedrigeren Schmelzpunkt und einen höheren Siedepunkt als das Wasser (WA) hat und
• wobei bei Erreichen der Stratosphäre die Temperatur in der Messzelle (MZ) über dem Schmelzpunkt oder unter dem Siedepunkt der wässrigen Lösung (WL) durch die thermodynamisch passiv bewirkte Zuführung oder den Entzug von Energie aus der wässrigen Reaktionslösung (WL) durch das Wasser (WA) bei dessen früherem Phasenübergang vor Erreichen des Tripelpunktes der wässrigen Lösung stabilisiert wird.

2. Ozonsonde nach Anspruch 1
**dadurch gekennzeichnet, dass**
die Wassermenge zur Befüllung des Gefäßes (GE) 25 g bis 50 g und die Grundfläche des Gefäßes (GE) 10 cm² bis 20 cm² beträgt.

3. Ozonsonde nach einem der Ansprüche 1 oder 2
**dadurch gekennzeichnet, dass**
das Gefäß (GE) nach oben vollständig offen ist.

4. Ozonsonde nach einem der Ansprüche 1 oder 2
**dadurch gekennzeichnet, dass**
das Gefäß (GE) einen Deckel mit einer kleinen Öffnung oder einem kurzen Entlüftungsschlauch (ES) aufweist.

5. Ozonsonde nach einem der Ansprüche 1 bis 4
**dadurch gekennzeichnet, dass**
das Gefäß (GE) eine zumindest teilweise Füllung mit porösem Material (PM) aufweist.

6. Ozonsonde nach Anspruch 5
**dadurch gekennzeichnet, dass**
das poröse Material (PM) Zellstoff (ZS) ist.

7. Ozonsonde nach einem der Ansprüche 1 bis 6
**dadurch gekennzeichnet, dass**
das Material des Gefäßes (GE) Teflon ist.

8. Ozonsonde nach einem der Ansprüche 1 bis 6
**dadurch gekennzeichnet, dass**
das Material des Gefäßes (GE) Polyethylen ist.

9. Ozonsonde nach einem der Ansprüche 1 bis 6
**dadurch gekennzeichnet, dass**
das Material des Gefäßes (GE) wasserdicht beschichtete Pappe ist.

## Claims

1. Ozone probe (OS) for in situ measurement of stratospheric ozone concentration profiles using balloon ascents, comprising a measurement cell (MZ) with an aqueous reaction solution (WL) with melting and boiling points depending on its method-related salt content, a pump (PU) for conducting ambient air (UL) through the aqueous reaction solution (WL), a current supply (SV), a device for registering the reaction current and transmitting the data (RD) and a support device (TV) for the arrangement of all the components,
**characterised in that**
the measurement cell (MZ) is surrounded by a hydrothermal buffer in the form of a vessel (GE) filled with water (WA),
• wherein the vessel (GE) completely surrounds the measurement cell (MZ), and the level of the water (WA) in the vessel (GE) at least reaches the level of the aqueous reaction solution (WL) in the measurement cell (MZ),
• wherein the aqueous reaction solution (WL) has a lower melting point and a higher boiling point than the water (WA) because of its method-related salt content, and
• wherein, when the stratosphere is reached, the temperature in the measurement cell (MZ) is stabilised above the melting point or below the boiling point of the aqueous solution (WL) because of the thermodynamically passively induced supply or withdrawal of energy from the aqueous reaction solution (WL) by the water (WA) during its earlier phase transition before reaching the triple point of the aqueous solution.

2. Ozone probe according to Claim 1,
**characterised in that**
the amount of water for filling the vessel (GE) is 25 g to 50 g, and the base area of the vessel (GE) is 10 cm² to 20 cm².

3. Ozone probe according to one of Claims 1 or 2,
**characterised in that**
the vessel (GE) is completely open at the top.

4. Ozone probe according to one of Claims 1 or 2,
**characterised in that**
the vessel (GE) has a lid with a small opening or a short ventilation hose (ES).

5. Ozone probe according to one of Claims 1 to 4,
**characterised in that**
the vessel (GE) has an at least partial filing with porous material (PM).

6. Ozone probe according to Claim 5,
**characterised in that**
the porous material (PM) is cellulose (ZS).

7. Ozone probe according to one of Claims 1 to 6,
**characterised in that**
the material of the vessel (GE) is Teflon.

8. Ozone probe according to one of Claims 1 to 6,
**characterised in that**
the material of the vessel (GE) is polyethylene.

9. Ozone probe according to one of Claims 1 to 6,
**characterised in that**
the material of the vessel (GE) is waterproof-coated cardboard.

## Revendications

1. Sonde d'ozone (OS) pour la mesure in situ de profils de concentration d'ozone stratosphériques par ballon dirigeable, comprenant une cellule de mesure (MZ) avec une solution auqueuse de réaction (WL), avec un point de fusion et d'ébullition dépendant de sa teneur en sel selon le procédé, une pompe (PU) pour la circulation d'air ambiant (UL) à travers la solution auqueuse de réaction (WL), une alimentation électrique (SV), un dispositif pour l'enregistrement du flux de réaction et la transmission des données (RD), ainsi qu'un dispositif de support (TV) pour la disposition de tous les composants,
**caractérisée en ce que**
la cellule de mesure (MZ) est entourée d'un récipient (GE) rempli d'eau (WA) en tant que tampon hydrothermique, où
• le récipient (GE) entoure entièrement la cellule de mesure (MZ) et le niveau d'eau (WA) dans le récipient (GE) atteint au moins le niveau de la solution aqueuse de réaction (WL) dans la cellule de mesure (MZ),
• la solution aqueuse de réaction (WL) présente un point de fusion inférieur et un point d'ébullition supérieur à ceux de l'eau (WA), de par sa teneur en sel selon le procédé, et où
• lors de l'atteinte de la stratosphère, la température dans la cellule de mesure (MZ) est stabilisée au-dessus du point de fusion ou en-dessous du point d'ébullition de la solution auqueuse (WL), du fait de l'alimentation en énergie, provoquée de façon thermodynamiquement passive, ou de l'évacuation d'énergie à partir de solution aqueuse de réaction (WL) par l'eau (WA), lors du changement de phase antérieur de celle-ci avant l'atteinte du triple point de la solution aqueuse.

2. Sonde d'ozone selon la revendication 1
**caractérisée en ce que**
la quantité d'eau nécessaire au remplissage du récipient (GE) est de 25 g à 50 g et la surface de base du récipient (GE) mesure entre 10 _{CM}² et 20 cm².

3. Sonde d'ozone selon l'une des revendications 1 ou 2,
**caractérisée en ce que**
le récipient (GE) est entièrement ouvert vers le haut.

4. Sonde d'ozone selon l'une des revendications 1 ou 2,
**caractérisée en ce que**
le récipient (GE) comporte un couvercle avec une petite ouverture ou un tuyau d'aération court (ES).

5. Sonde d'ozone selon l'une des revendications 1 à 4,
**caractérisée en ce que**
le récipient (GE) est au moins partiellement rempli d'une matière poreuse (PM).

6. Sonde d'ozone selon la revendication 5,
**caractérisée en ce que**
la matière poreuse (PM) est de la pâte de cellulose (ZS).

7. Sonde d'ozone selon l'une des revendications 1 à 6,
**caractérisée en ce que**
le matériau du récipient (GE) est du téflon.

8. Sonde d'ozone selon l'une des revendications 1 à 6,
**caractérisée en ce que**
le matériau du récipient (GE) est du polyèthylène.

9. Sonde d'ozone selon l'une des revendications 1 à 6,
**caractérisée en ce que**
le matériau du récipient (GE) est un carton à revêtement étanche à l'eau.
